(19) Europäisches Patentamt
European Patent Office
Office européen des brevets

(11) EP 0 723 547 B1

# (12) EUROPEAN PATENT SPECIFICATION

(45) Date of publication and mention of the grant of the patent:
**23.08.2000 Bulletin 2000/34**

(21) Application number: **94928666.0**

(22) Date of filing: **26.09.1994**

(51) Int. Cl.7: **A61K 49/00**, C07F 5/02, C07D 487/22, C07K 14/795, A61K 31/40

(86) International application number:
**PCT/US94/10863**

(87) International publication number:
**WO 95/09856 (13.04.1995 Gazette 1995/16)**

## (54) BOROANTED METALLOPORPHYRINS AND DIAGNOSTIC METHODS

BORIERTE METALLOPORPHYRINE UND DIAGNOSTISCHE VERFAHREN

METALLOPORPHYRINES DE BORE ET LEURS UTILISATIONS DIAGNOSTIQUES

(84) Designated Contracting States:
**BE CH DE FR GB IT LI NL**

(30) Priority: **01.10.1993 US 130302**

(43) Date of publication of application:
**31.07.1996 Bulletin 1996/31**

(73) Proprietor:
**The Regents of the University of California**
**Oakland, CA 94607-5200 (US)**

(72) Inventors:
- **KAHL, Stephen, B.**
  **Portola Valley, CA 94028 (US)**
- **KOO, Myoung-Seo**
  **San Francisco, CA 94122 (US)**

(74) Representative:
**Goldin, Douglas Michael**
**J.A. KEMP & CO.**
**14 South Square**
**Gray's Inn**
**London WC1R 5LX (GB)**

(56) References cited:

**EP-A- 0 350 948**
**WO-A-92/09610**
**WO-A-94/05285**
**US-A- 4 959 356**
**US-A- 5 262 532**

- **DECAMP, DIANNE L. ET AL: "Specific inhibition of HIV-1 protease by boronated porphyrins" J. MED. CHEM. (1992), 35(18), 3426-8 CODEN: JMCMAR;ISSN: 0022-2623, 1992, XP002048823**
- **HILL J S ET AL: "SELECTIVE TUMOR UPTAKE OF A BORONATED PORPHYRIN IN AN ANIMAL MODEL OF CEREBRAL GLIOMA" PROCEEDINGS OF THE NATIONAL ACADEMY OF SCIENCES OF USA, vol. 89, March 1992, pages 1785-1789, XP000615925**
- **NGUYEN TIEN ET AL: "Intracellular distribution of various boron compounds for use in boron neutron capture therapy" BIOCHEM. PHARMACOL. (1993), 45(1), 147-55 CODEN: BCPCA6;ISSN: 0006-2952, 1993, XP002048824**
- **M. MIURA ET AL.: TETRAHEDRON LETTERS, vol. 31, no. 16, 1990, OXFORD GB, pages 2247-2250, XP002048825**
- **TETRAHEDRON, Volume 38, No. 15, issued 1982, K.M. SMITH et al., "The NMR Spectra Porphyrins-19. C and Proton NMR Spectra of Metal-Free Porphyrins with the Type-IX Substituent Orientation, and of their Zinc(II) Complexes", pages 2441-2449.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 723 547 B1

## Description

The present invention generally relates to diagnostic uses of porphyrins.

U S Patent 4,959,356, issued September 25, 1990, inventors Miura and Gabel, describes boronated porphyrin compounds for use in boron neutron capture therapy (BNCT). Similar compounds are discussed in an article by Miura et al. *Tetrahedron Letters*, Vol. 31, No. 16, pp. 2247-2250 (1990). The boronated porphyrins described by Miura have vinyl carborane moieties and are reported to be water insoluble. Thus the borane cages must be opened to obtain water solubility. But by opening those borane cages, one encounters significantly more toxicity for the compounds. Moreover, the resultant open-cage compounds are still not sufficiently water soluble to enable administration without the use of adjuvant substances (e.g., polyethylene glycol). Also the compounds which Miura et al. describe are (at most) 19% boron by weight in the physiologically useful ($K^+$-salt) form. This is a disadvantage since limiting human doses may well be determined by the amount of porphyrin unit doses which may be tolerated.

In addition to neutron capture therapy (NCT) generally and boron neutron capture therapy (BNCT) more specifically, additional uses of porphyrins in cancer therapies are those therapeutic strategies generally referred to as photodynamic therapy (PDT). A review article by Delaney and Glatstein in *Comprehensive Therapy*, pp. 43-55 (May 1988) describes this therapeutic strategy where a light-activated photosynthesizer can interact with ground state molecular oxygen to yield reactive oxygen species (via singlet oxygen). Since porphyrins of many structural types localize in a wide variety of malignant tumors, this localization has formed the basis for treatment of at least 3000 patients in the United States alone (twice that worldwide) over the past several years through PDT. Complete response (disappearance of tumor or biopsy proven) has occurred in a high percentage of patients in relatively advanced stages of skin, bladder, and lung-cancers, and cancers of the reproductive system through photodynamic therapy.

U.S. Patent 5,109,016, issued April 28, 1992, inventors Dixon et al., describes compositions said to inhibit replication of human immunodeficiency virus by porphyrin and certain porphyrin-like compounds. These compounds were tested for inhibition of reverse transcriptase as a screening method to determine inhibition of HIV; however, during the 8th International Conference on AIDS, held in late July, 1992, in Amsterdam, some presentations suggested that many drugs that showed initial promise against HIV-1 have been found to have serious shortcomings because HIV-1 rapidly becomes resistant to the so-called "non-nucleoside reverse transcriptase inhibitors." See, *C&EN*, 70:34, pp. 26-31 (August 24, 1992). As an example of a non-nucleoside derivative that binds to a site other than the active site (non-competitive inhibition), see Pauwels et al., *Nature, 343*, pp. 470-474 (1990); however, resistance develops rapidly to this derivative.

DeCamp et al (1992) J. Med. Chem. 35 3426-3428 dicloses that boronated porphyrins inhibit HIV-1 protease. WO 92/09610 describes boronated porphyrins useful in BNCT and PDT. EP-A-350948 describes metalloporphyrin commpounds useful as therapeutic and diagnostic agents for malignant tumors, as tumor markers and as agents for use in missile therapy of cancers. US Patent No. 5,262,532 describes porphyrin-complex compounds useful as contract agents in magnetic resonance imaging (MRI).

### Summary of the Invention

The invention provides the use of a biologically active, porphyrin-based compound for the manufacture of a composition for diagnosis of a tumor by magnetic resonance imaging, said compound having the structure:

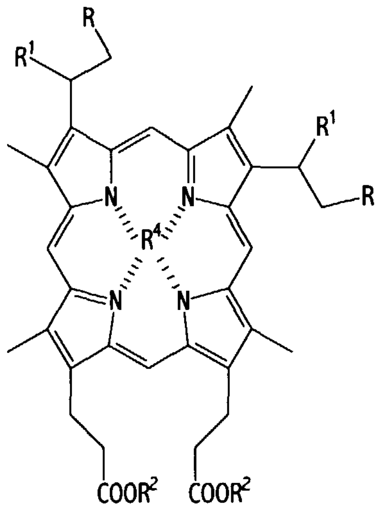

where each of R and $R^1$ is selected from -H, -OH and

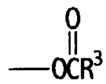

and at least one of R and $R^1$ is

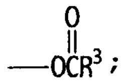

$R^3$ is a carborane; $R^2$ is -H, an alkyl having 1 to about 7 carbon atoms, an aryl having up to 7 carbon atoms, or forms a physiologically acceptable salt; and $R^4$ is a transition metal.

Particularly preferred as the porphyrin compound is the manganese chelate of the tetrakis-carborane carboxylate ester of 2,4-(α,β-dihydroxyl-ethyl)deuteroporphyrin IX. This particularly preferred porphyrin compound, which we call "Mn-BOPP", can enhance magnetic resonance imaging (MRI) contrast between tumor and normal tissue, and has been tolerated in mice at doses as high as 100 mg/kg.

Brief Description of the Figure

Figure 1 graphically illustrates the percent difference of MRI signal intensity between tumor and normal brain tissue before and after administration of a preferred compound usable in the invention.

Detailed Description of the Preferred Embodiments

Broadly, the invention uses compositions where the compound being used can be viewed as consisting of or built from a porphine precursor, whose basic structure is illustrated as Formula P:

FORMULA P

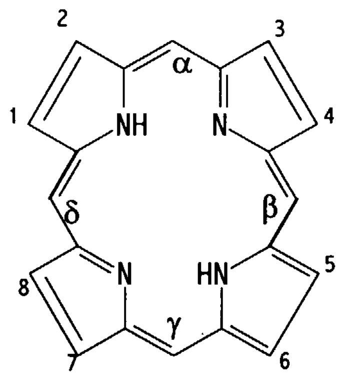

Porphine, of course, is the parent (or core) substance of the porphyrins, which have side chains and alkyl groups substituted for some hydrogens in the porphine pyrrole rings. Compounds used in the invention have substituents on the pyrrole rings of porphine at pyrrole ring positions 2 and 4.

Particularly preferred compounds are where $R^3$ is a *closo*-carborane, and $R^4$ is Mn(III), Cu(II) or Co(II).

The presence of the $R^4$ transition metal assists in stabilizing the molecules against, for example, sensitivity to light. The presence of the $R^4$ transition metal, particularly manganese, also provides the function as a contrast agent for MR imaging. This is important because conventional manipulation of pulse sequences frequently does not provide clear differentiation between tumor and normal tissue in MR imaging. Most contrast agents currently under investigation do not localize specifically and selectively in tumors, but rather remain solubilized in blood or in extra vascular compartments.

Formulations may be prepared having the desired degree of purity with optional physiologically acceptable carriers, excipients or stabilizers. Acceptable carriers, excipients or stabilizers are nontoxic to recipients at the dosages and concentrations employed when administered, and include buffers such as phosphate, citrate, and other organic acids; anti-oxidants including ascorbic acid; low molecular weight (less than about 10 residues) polypeptides; and proteins, such as serum albumin, gelatin and immunoglobulins. Other components can include glycine, blutamine, asparagine, arginine, or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween, Pluronics or PEG.

Conjugates, or complexes, of the compounds with lipoprotein are also useful and can be simply prepared for delivery of a compoound using low density lipoprotein as the carrier agent. Either of two methods of complexation are preferred. For the first, human low desnsity lipoprotein, isolated from fresh human plasma by preparative density gradient ultracentrifugation, is treated by the method described by Kahl and Callaway, *Strahlenther Onkol*. 165:137 (1989), incorporated by reference. In brief, this method involves removal of the native cholesterol ester core of LDL by a published procedure, Krieger et al., *Biol. Chem*., 259:3845 (1979), also incorporated by reference, incubation of the delipidated LDL with a solution of BOPP-dimethyl ester in $CCl_4$ (6 mg/200 μL), removal of the $CCl_4$ by nitrogen flow, and solubilization of the reconstituted LDL in 10mM tricine (pH 8.6). This procedure results in LDL particles containing approximately 300 molecules of BOPP-dimethyl ester per LDL particle. LDL reconstituted in this manner behaves in a fashion similar to native LDL and enters cells by receptor-mediated endocytosis.

A second method is simpler, but results in fewer molecules of the compound per LDL particle. Human plasma is incubated at 37°C with an aqueous solution of BOPP (in the potassium salt form; 4μM) for 30 minutes. The plasma lipoprotein fractions are then separated by density gradient ultracentrifugation. Approximately 25% of the administered porphyrin is found bound to the LDL fraction, 20% to the VLDL, and 55% to the HDL fraction.

Compounds with boron cage systems in which one or more carbon atoms is present as an integral part of an electron-delocalized boron framework are given the general name "carboranes", which term includes both closed polyhedra and open-cage structures. Carboranes are distinct from other organoboron species, such as the alkyl boranes, because the carbon(s) are part of the cage itself rather than present as a ligand. An early monograph on these electron-deficient boron cage compounds is by Grimes, *Carboranes*, Academic Press (1970), which describes nomenclature, structure, synthesis, and properties of carboranes, including those of interest for this invention. There are a

series of stable 12-atom polyhedral boron cage systems that have been isolated in at least three isomeric forms. Two particularly preferred cage system isomers for this invention are the 1,2-$C_2B_{10}H_{12}$ isomer and the 1,7-$C_2B_{10}H_{12}$ isomer (the latter somethimes represented as "$HCB_{10}H_{10}CH$").

Particularly preferred for embodiments of the invention are wherein $R^3$ is a *closo*-carborane, and most particularly preferred is the 1,2-icosahedral isomer or 1,7-icosahedral isomer, whether substituted or unsubstituted. An illustrative substituent is, for example, a carboxyl group, which may be desirable to aid in solubilizing the porphyrin compound. The physical properties of the 1,2-icosahedral isomer and the 1,7-icosahedral isomer are similar.

The *closo*-carboranes are particularly preferred because the open-cage carboranes lead to significantly increased toxicity of what can otherwise be substantially non-toxic compounds. Closed-cage and open-cage systems are typically designated by the prefixes "*closo*-" and "nido-," respectively.

Conversion of the bis-glycol substituents to tetra-ester is surprising due to the size and steric hindrance considerations for preferred moieties. To obtain a di-ester carborane (such as with $R^1$ at each of the 2 and 4 porphyrin ring positions as hydroxyl or hydrogen), then typical acylation agents, such as pyridine or trimethylamine can be used. To make the tetra-esters, such as in preparing a particularly preferred compound we have coined as "BOPP", then the conversion of bis-glycol critically depends upon the use of p-dimethylaminopyridine (DMAP) as a hyper-acylation reagent.

The BOPP compound is especially useful where a stable, quite water soluble, substantially non-toxic compound with a large number of boron atoms (40) is desired. We have designated the BOPP compound, tetra-carborane carboxylate ester of 2,4-di(α,β-dihydroxyethyl)deuteroporphyrin (IX), as "compound 1".

Porphyrin compositions as described for use in accordance with this invention are substantially bio-available.

Aspects of the invention will now be exemplified by the following examples, which are understood to be illustrative and not limiting.

**EXAMPLE 1**

Synthesis of bis-glycol porphyrin dimethyl ester

Dried protoporphyrin dimethyl ester (4.4 g) was dissolved in 1.6 L of dioxane and 3.0 mL of pyridine in the dark. This solution was thoroughly bubbled with argon to remove oxygen. Osmium tetroxide (4.0 g) was dissolved in 200 ml argon-degassed diethyl ether and added to the dioxane-porphyrin solution. The solution was well stirred under Ar in the dark for 24 hours. Sodium sulfite (8.8 g) was dissolved in 160 mL distilled, argon-degassed water and added to the solution. The reaction was heated to 75°C on a steam bath for 6 hours. The reaction solution was cooled to 53-55°C and filtered quickly through a 1.5 L medium fritted funnel and washed with a small portion of dioxane. Filtrate was evaporated *in vacuo* and then 200 mL and 750 mL of water was added with stirring to crystallize the product. The crystals were filtered through a 1.5 L medium fritted funnel and washed with 100 mL water. The filtered solid was suspended in 200 mL, 15% methanol/methylene chloride and 450 mL hexane added with stirring to complete crystallization. The procedure was repeated to remove impurities if necessary.

**EXAMPLE 2**

Synthesis of 2,4-bis-[α,β-(1,2-dicarbaclosododecaborane carboxy)ethyl]deuteroporphyrin (IX) dimethyl

Carborane carboxylic acid was synthesized from 1,2-$B_{10}C_2H_{12}$ by the method of Zakharkin et al., Akad. Nauk SSSR, *Ser. Khim*., p. 1376 (1967) and Zakharkin et al., *Tet. Lett*., p. 1147 (1964), incorporated by reference. Briefly, this method involves treatment of the o-carborane with one equivalent of n-butyl-lithium in benzene to produce the mono-lithio compound. This species is reacted with $CO_2$ to give the lithium salt of the carboxylate which gives the free carboxylic acid on acidification. Conversion to the acid chloride is also by the method of Zakharkin et al. using $PCl_5$ in toluene. Vacuum distillation of the reaction mixture gives the carborane carbonyl chloride.

The bis-glycol porphyrin prepared as described by Example 1 (500 mg, 0.75 mmol) was dissolved in dry methylene chloride (200 mL), and the solution bubbled with argon. To the above solution o-carboranyl acid chloride (690 mg, 3.341 mmol) was added. The solution was stirred, and 4-dimethyl aminopyridine (DMAP) 371 mg, 3 mmol) was added to the solution. The solution was stirred at room temperature for one hour and poured into water. The organic layer was separated, washed with dilute hydrochloric acid three times, saturated sodium bicarbonate (3x), water (2x), and dried over sodium sulfate. Unreacted carborane carboxylic acid was removed from the bicarbonate washes by acidification and extraction with henna or diethyl ether. The solution was filtered and evaporated *in vacuo* to yield a crude product. Three spots were obtained on analytical TLC plate by developing with 100% methylene chloride. The top spot is the tetracarboranyl porphyrin dimethyl ester, and the two slower spots are tri- and di-carborane esters, respectively. The tetracarboranyl porphyrin dimethyl ester was separated by filtering the methylene chloride solution of the crude

products through a silica gel pad. The first mobile band was obtained by washing with 100% methylene chloride (110 mL) and evaporating to dryness. Crystallization from methylene chloride-hexane gave 849 mg. Isolated yield was 80-85%. We coined "BOPP" as a shorthand designation for the compound whose preparation has just been described (in the dimethyl ester form).

The conversion of bis-glycol to tetra-ester critically depends upon the use of p-dimethylaminopyridine (DMAP) as a hyper-acylation reagent and upon its stoichiometric relationship with bis-glycol and acid chloride. The rate of formation of tetra-ester product in the absence of nitrogen base is extremely slow, if it occurs at all. We have found that when pyridine or trimethylamine (two other frequently used acylation agents) were used rather than the DMAP reagent, then the rate of tetra-ester formation was very slow when compared to use of DMAP. However, when one wishes to prepare the diester form rather than the tetra-ester, then these other acylation agents (such as TEA) should be used rather than DMAP.

**EXAMPLE 3**

Synthesis of 2,4-bis-[α,β-(1,2-dicarbaclosododecaborane carboxy)ethyl]deuteroporphyrin (IX)

To a solution of the tetracarboranylporphyrin dimethyl ester prepared as described in Example 2 (300 mg, 0.224 mmol) in 100 mL ether was added 25% hydrochloric acid (100 mL). The solution was stirred at room temperature overnight. The solution was washed with copious water (to dilute acid). The ether layer was separated, dried over sodium sulfate and evaporated *in vacuo* to give the tetracarboranyl porphyrin diacid compound we designate in shorthand as "BOPP free acid" Quantitative yield.

**EXAMPLE 4A**

Synthesis of 2,4-bis-[α,β-(1,2-dicarbaclosododecaborane carboxy)ethyl]deuteroporphyrin dipotassium salt (IX)

The tetracarboranyl porphyrin diacid, or "BOPP free acid" prepared as described in Example 3, (150 mg) was dissolved in 20 mL THF and 15 mL water was added. The solution was passed through 1x10 cm cation exchange resin, 200-400 dry mesh. The eluate was evaporated to increase the ratio of water (up to 60/40 water/THF) and passed through ion exchange resin again. The final eluate was evaporated to dryness *in vacuo*. The resulting dipotassium salt is well solubilized in water. We designated this compound as "BOPP". We now prefer using acetone instead of THF.

**EXAMPLE 4B**

Synthesis of 1.7 Isomer

The 1,7 *closo*-carborane isomer of BOPP was prepared in the same manner using 1,7-carborane carbonyl chloride, but with the following difference. Thermal isomerization of the 1,2-carborane to the 1,7-carborane occurs at 450-500°C. See Grafstein and Dvorak, *Inorganic Chemistry*, 2:1128 (1963). Yields in this process approach 95% when a flow through a pyrolysis system is used in conjunction with an inert carrier gas. This approach is preferred for the conversion of $^{10}$B-enriched 1,2 isomer to the 1,7 isomer.

Other sterically hindered organic acid chlorides were used to incorporate desired $R^3$ moieties in an analogous manner to Examples 1-4A. Characterization in all cases was by mass spectrometry, proton magnetic resonance, and UV-visible spectroscopy.

**EXAMPLE 5**

Evidence in support of the structures prepared in Examples 2-4A came from mass spectrometric and spectroscopic sources. The LSIMS mass spectrum of BOPP-dimethyl ester in a tetraethylene glycol matrix shows a molecular ion cluster ($MH^+$) at nominal mass 1341 corresponding to the formula $C_{48}H_{83}B_{40}N_4O_{12}$. The shape of the theoretical molecular ion cluster of this formula is nearly identical to the 15-peak ion cluster observed at 1341. Similarly, LSIMS of the BOPP free acid produces a molecular ion cluster at nominal mass 1321 corresponding to the formula $C_{46}H_{79}B_{40}N_{40}O_{12}$ and whose shape is almost identical to the theoretical molecular ion cluster. In both mass spectra, four successive losses of $B_{10}H_{11}C_3O_2$ fragments are observed corresponding to loss of the carborane carboxylate. The visible spectrum of BOPP-dimethyl ester in $CH_2Cl_2$ (10 μM) consists of peaks at 404 (Soret) 502, 536, 572, and 624 nm.

A summary of the 300 MHz proton N.M.R. data for BOPP-dimethyl ester is presented in Table 1.

**TABLE 1***

| δ | multiplicity | assignments |
|---|---|---|
| 10.27, 10.23, 10.16, 10.14 | s, 4H | meso H |
| 7.68 | m, 2H | α CH |
| 5.68; 4.99 | m, 4H | ß $CH_2$ |
| 4.41 | m, 4H | Por-$CH_2$ |
| 4.17; 4.10 | s, 4H | carborane CH |
| 3.81; 3.80 | s, 6H | $CO_2CH_3$ |
| 3.68; 3.64<br>3.63; 3.61 | s, 12H | ß-$CH_3$ |
| 3.30 | m, 4H | -$CH_2CO_2R$ |
| -3.65 | s, 2H | NH |

* N.M.R. spectrum was recorded in $CDCl_3$ (ca. $5x10^{-3}$ M) at 300 MHz at ambient temperature with TMS internal reference.

The lack of a molecular $C_2$ axis is clearly demonstrated by the presence of four, distinct resonances for the meso-H and β-$CH_3$ groups. This makes difficult a detailed analysis of the spectrum, especially the conformation of the carboranyl ester-bearing side chains. Nevertheless, several features are apparent. At least two distinct and equivalent carborane CH environments are present, perhaps a reflection of the primary and secondary alcohol ester functions. Three resonances are assigned to the two-carbon side chain protons, a chiral methine ($H_\alpha$), and pro-chiral methylene ($H_\beta$). The $H_\alpha$ assigned to 7.68 ppm is strongly deshielded by virtue of its being bound to a carbon bearing two strongly deshielding groups: the porphyrin (ring current) and carboranyl acyl (σ effects). The two $H_\beta$ resonances have significant (0.7 ppm) chemical shift differences which might arise if the most stable configuration forces one of the $H_\beta$ protons into a position over the porphyrin ring when it is subject to ring current deshielding relative to the other.

There are three possible configurations for the groups bound to the ethyl side chain, two gauche and one anti. Computer modeling based on the crystal structure of mesoporphyrin IX dimethyl ester suggests that the anti-configuration should offer the least amount of steric hindrance for all functional groups. The α-carborane ester points down at an angle of about 45° and away from the porphyrin while the β-ester points up and over the porphyrin. This configuration also places one of the pro-chiral $H_\beta$ protons in a pocket formed by the porphyrin plane, a meso-H and the $O_\alpha$, giving rise to its potential deshielding relative to the other $H_\beta$. However, one gauche configuration also provides some side chain flexibility and deshielding of one $H_\beta$ from a similar pocket. In the remaining gauche form, all J-J coupling constants should be large and modeling indicates significant steric hindrance for the $O_\beta$.

**EXAMPLE 6A**

In a manner analogous to that described by the just described examples, a number of compounds were prepared where Table 2 gives the R, $R^1$, and $R^4$ moieties.

TABLE 2

| Compound | R | $R^1$ | $R^4$ |
|---|---|---|---|
| 1 | $OCOB_{10}H_{11}C_2$ | Same as R | $H_2$ |
| 2 | H | $OCOB_{10}H_{11}C_2$ | $H_2$ |
| 3 | $OCOB_{10}H_{11}C_2$ | Same as R | Mn(III) |
| 4 | $OCOB_{10}H_{14}C_3$ | Same as R | $H_2$ |
| 5 | $OCOB_{10}H_{11}C_2$ | Same as R | Cu(II) |
| 6 | $OCOB_{10}H_{11}C_2$ | Same as R | Co(II) |
| 7 | OCO(adamantoyl) | Same as R | $H_2$ |
| 8 | OCOp-[$(CH_3)_2$N]benzoyl | Same as R | $H_2$ |
| 9 | meta-$OCOB_{10}H_{11}C_2$ | Same as R | $H_2$ |
| 10 | $OCOC_6H_5$ | Same as R | $H_2$ |
| 11 | OCO(β-naphthoyl) | Same as R | $H_2$ |
| 12 | OH | OH | $H_2$ |

Compounds 3, 5 and 6 are compounds that may be used in the invention, and compounds 1, 2, 4 and 7 to 12 are comparative compounds.

Compound 2 was prepared in the dimethyl ester form as described by Example 2 and converted to the free acid as by Example 3.

Compounds 3, 5, and 6 are metalloporphyrin derivatives of BOPP prepared by the general procedure outlined below (and also exemplified in Example 6B).

BOPP dimethyl ester (130 mg/0.099 mmol) was dissolved in glacial acetic acid (9 ml) and pulverized anhydrous manganese (II) acetate (50 mg; 0.289 mmol) was added. The vessel was loosely stoppered and the solution stirred in the dark at 20°C. The course of reaction was followed spectrophotometrically by observing the disappearance of the red fluorescence characteristic of free base porphyrin. When all traces of this red fluorescence had disappeared (20 h), ether (125 ml) was added to the solution. The ether solution was washed with an equal volume of water (3x), dried over sodium sulfate, and evaporated *in vacuo*. The solid product was redissolved in ether (30 ml) and 25% aqueous HCl (30 ml) added. After stirring overnight at 20°C, the mixture was poured into ether (200 ml) and washed four times with water (500 ml). The ether layer was again dried, filtered, and evaporated *in vacuo* to yield pure product (166 mg, 75.1% yield).

The water soluble Mn(III) porphyrin dipotassium salt is estimated to contain 5% water by weight. The absorption spectrum of this compound showed no evidence of free-base porphyrin and consisted of peaks at 368 nm ($\log \varepsilon = 4.82$), 418 nm (sh) (4.36), 460 nm (4.60), 546 nm (3.98), and 579 nm (sh) (3.82). The Cu(II) (compound 5) and Co(II) (compound 6) derivatives were prepared in a similar fashion to 3 using the appropriate metal salt. Their UV-Vis absorption spectra were similar to that of compound 3 and were characteristic of metalloporphyrins of the hematoporphyrin class. As with 3, no evidence of unmetallated free base porphyrin was observed in the UV-Vis spectra.

## EXAMPLE 6B

A preparation of Mn-BOPP was used in the tumor enhancement studies reported in Example 7. To 335 mg (0.254 mmol) of the tetracarboranyl porphyrin diacid (BOPP diacid) dissolved in 20 mL of glacial acetic acid was added 110 mg (0.636 mmol) of anhydrous manganese diacetate. The flask was loosely stopped and the solution stirred magnetically in the dark at 23°C. The course of the reaction was followed spectrophotometrically. After 24 hours, no red fluorescence of the free-base porphyrin starting material was observed, and the glacial acetic acid was removed at 50°C in vacuo. The residue was dissolved in 300 mL, of ether and washed with 300 mL, of water four times. The ether solution was dried over sodium sulfate, filtered, and evaporated to give the product in quantitative yield. The dipotassium salt was prepared by dissolving the Mn-BOPP diacid in a 70% solution of tetrahydrofuran and water and passing it through a column containing Dowex 50 x 2-400 cation exchange resin in the $K^+$ form. The colored eluate was evaporated to dryness, redissolved in a 30% solution of tetrahydrofuran and water, and passed through a column containing freshly generated resin. (We now prefer acetone instead of THF.) The eluate was evaporated in vacuo to give a quantitative yield

of the water-soluble dipotassium salt form of Mn-BOPP. This material contains approximately 3-5% water by weight. The anhydrous molecular weight of this material (with acetate as the counter ion) is 1,517.6. The purity is more than 99.9%.

## EXAMPLE 7

We will sometimes refer to compound 3 as "Mn-BOPP". This compound was prepared as described by Example 6B. The rat brain tumor cell line 9L (designated 9L-72) was obtained from Denise Deen, Ph.D., of the Brain Tumor Research Center, University of California, San Francisco. Cells were maintained in Dulbecco-modified minimum essential medium containing 5% fetal bovine serum and 5% calf serum. Cultures were renewed from frozen stocks approximately monthly.

Fischer 344 rats (Harlan-Sprague-Dawley, Indianapolis, Indiana) (three to five for each experiment) weighing approximately 300 g were anesthetized by intramuscular injection of ketamine and xylazine (66.7 and 6.7 mg/kg body weight, respectively). After induction of surgical anesthesia, animals were placed in a stereotaxic frame. A midline incision was made, and the scalp was reflected. A small burr hole was drilled 2 mm anterior and 2.4 mm lateral to the bregma. A sharp 26-gauge Hamilton syringe was lowered to a depth of 4.5 mm, and 9L cells ($5 \times 10^4$ cells in 1.5 µL of saline) were injected over a period of two minutes. After injection, a small fragment of gelatin sponge (Gelfoam; Upjohn, Kalamazoo, Michigan) was placed in the burr hole and the scalp was sutured. All animal work was performed according to protocols reviewed and approved by the Laboratory Animal Care Committee, State University of New York, Buffalo.

MR imaging was performed on a Signa Advantage 1.5-T whole-body clinical imager (GE Medical Systems, Milwaukee, Wisconsin) with a custom-built transceiver rat head coil and stereotaxic head holder (detailed information to be published separately). All images were obtained with a T1-weighted spin-echo sequence, with a TR of 400 msec, a TE of 20 msec (400/20), a 3-mm section thickness, an 8-cm field of view, and a 256 x 256 matrix.

The ultraviolet-visible spectral data for the Mn-BOPP shows the Soret band at 368 nm ($\varepsilon = 5.9 \times 10^4$). Mn-BOPP was dissolved in 0.9% sodium chloride to a concentration of 10 mg/mL and administered intravenously to the rats at a dose of 0.02 mmol/kg. Rats with 12-day-old tumors were anesthetized with an intramuscular injection of ketamine and xylazine (66.7 and 6.7 mg/kg body weight, respectively), and their heads were positioned in the head holder. Initially, a sagittal locator section was obtained. The coronal sections were graphically prescribed from the sagittal locator. The coronal plane is defined as the longitudinal section dividing the body into dorsal and ventral parts (also referred as the horizontal plane). The rats were then imaged in the coronal plane before and within 5 minutes after the administration of the metalloporphyrin compounds. Subsequent images were also obtained at various times over a period of 2-4 days.

Research software on the Signa imager provides values for the mean signal intensity and the standard deviation measured from a user-defined region of interest of a specific anatomic area on the images. Signal intensities of the tumor region and the corresponding normal brain region in the right hemisphere were measured on all images. The difference in signal intensity between the tumor and normal brain was expressed as $(SI_{tumor} - SI_{normal})SI_{normal} \times 100$, the percent contrast enhancement of the tumor.

The T1 values of Mn-BOPP in water were measured at 0.25 T. The values for the molar relaxivity of Mn-BOPP evaluated from the slopes of the plot of relaxation rates (1/T1) versus concentrations, are 3.60 and 4.43 $L \cdot mmol^{-1} \cdot sec^{-1}$ at 25°C and 37°C, respectively.

Rats were imaged before and after administration of Mn-BOPP. The glioma was apparent on the precontrast control image as a slightly hyperintense area semicircumscribed anteriorly by a hypointense crescent in the frontal region of the left hemisphere. The posterior margin of the tumor was nearly isointense to normal tissue. The immediate postcontrast image showed the tumor as slightly enhanced and the blood pool was substantially enhanced, most notably along the midline and medial margin of the tumor and extending anteriorly between the hypointense border of the tumor and posteriorly to the olfactory bulb. The subsequent image obtained at 24, 40, 69, and 92 hours showed that Mn-BOPP was retained in the tumor and that the tumor mass appeared homogeneously enhanced. Mn-BOPP is able to delineate clearly an apparent tumor boundary and to markedly enhance contrast between tumor and normal brain. The plot of percent difference in signal intensity between tumor and normal brain tissue versus time is shown in Fig. 1. The maximal enhancement of contrast between tumor and normal brain with Mn-BOPP was observed at 24 hours after injection.

These results clearly demonstrate the accumulation and selective retention of manganese in tumor. Compound 3 is therefore an effective tumor-specific contrast agent.

Compound 3 was effective at very low doses. For rats injected with Mn-BOPP at a dose of 0.02 mmol/kg the porphyrin was observed in the urine as a early as 15 minutes after injection. However, despite the low dose, the tumors were markedly enhanced and achieved a level of contrast that clearly delineated tumor from normal brain tissue. This tumor enhancement was observed after about 15 minutes, although Mn-BOPP was clearly seen in the blood pool of the brain within 5 minutes after injection.

## Claims

1. Use of a biologically active, porphyrin-based compound for the manufacture of a composition for diagnosis of a tumor by magnetic resonance imaging, said compound having the structure:

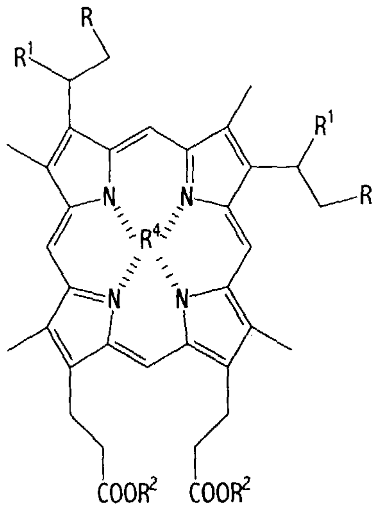

where each of R and $R^1$ is selected from -H, -OH and

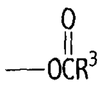

and at least one of R and $R^1$ is

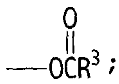

$R^3$ is a carborane; $R^2$ is -H, an alkyl having 1 to about 7 carbon atoms, an aryl having up to 7 carbon atoms, or forms a physiologically acceptable salt; and $R^4$ is a transition metal.

2. The use as in claim 1, wherein $R^4$ is manganese, copper or cobalt.

3. The use as in claim 1, wherein $R^4$ is manganese.

4. The use as in any one of the preceding claims, wherein $R^3$ is a *closo*-carborane.

5. The use as in claim 4, wherein the carborane is a substituted or unsubstituted 1,2-icosahedral isomer.

6. The use as in claim 4, wherein the carborane is a substituted or unsubstituted 1,7-icosahedral isomer.

7. The use as in any one of the preceding claims, wherein the compound is the manganese chelate of the tetrakis-carborane carboxylate ester of 2,4-(α,β-dihydroxyl-ethyl)deutero-porphyrin IX.

## Patentansprüche

1. Verwendung einer biologisch aktiven, auf Porphyrin basierenden Verbindung für die Herstellung einer Zugammensetzung für die Diagnose eines Tumors durch Kernspinresonanztomographie, wobei die Verbindung die folgende Struktur aufweist:

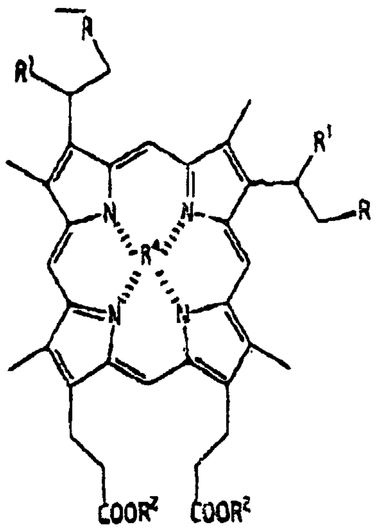

in der jeder von R und $R^1$ aus -H, -OH und

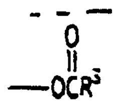

ausgewählt ist und mindestens einer von R und $R^1$

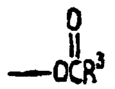

ist; $R^3$ ein Carboran ist; $R^2$ -H, ein Alkyl mit 1 bis ungefähr 7 Kohlenstoffatomen, ein Aryl mit bis zu 7 Kohlenstoffatomen ist oder ein physiologisch annehmbares Salz bildet; und $R^4$ ein Übergangemetall ist.

**2.** Verwendung nach Anspruch 1, wobei $R^4$ Mangan. Kupfer oder Cobalt ist.

**3.** Verwendung nach Anspruch 1, wobei $R^4$ Mangan ist.

**4.** Verwendung nach einem der vorangegangenen Ansprüche, wobei $R^3$ ein *closo*-Carboran ist.

**5.** Verwendung nach Anspruch 4, wobei das Carboran ein substituiertes oder unsubstituiertes 1,2-ikosaedrisches Isomer ist.

**6.** Verwendung nach Anspruch 4, wobei das Carboran ein substituiertes oder unsubstituiertes 1,7-ikosaedrisches Isomer ist.

**7.** Verwendung nach einem der vorangegangenen Ansprüche, wobei die Verbindung das Manganchelat des Tetrakiscarborancarboxylatesters von 2,4-(α,β-Dihydroxylethyl)deuteroporphyrin IX ist.

## Revendications

**1.** Emploi d'un composé biologiquement actif, à base de porphyrine, pour la préparation d'une composition destinée à servir au diagnostic d'une tumeur par imagerie par résonance magnétique, ledit composé présentant la structure suivante :

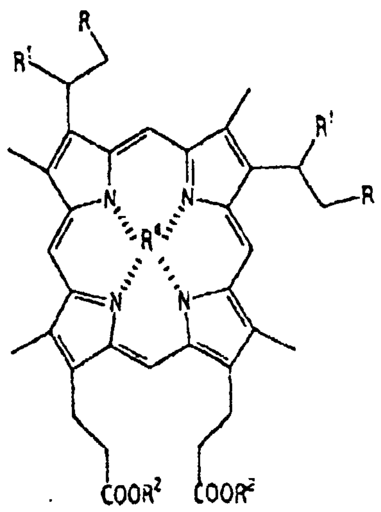

dans laquelle

chacun des symboles R et $R^1$ représente -H, -OH ou -OC-(O)$R^3$, mais au moins l'un des symboles R et $R^1$ représente -OC(O)$R^3$, $R^3$ représentant un reste de carborane ;
$R^2$ représente un atome d'hydrogène, un groupe alkyle comportant à peu près de 1 à 7 atomes de carbone ou un groupe aryle comportant jusqu'à 7 atomes de carbone, ou bien quelque chose qui forme un sel physiologiquement admissible ;
et $R^4$ représente un atome d'un métal de transition.

**2.** Emploi conforme à la revendication 1, dans lequel $R^4$ représente un atome de manganèse, de cuivre ou de cobalt.

**3.** Emploi conforme à la revendication 1, dans lequel $R^4$ représente un atome de manganèse.

**4.** Emploi conforme à l'une des revendications précédentes, dans lequel $R^3$ représente le reste d'un *closo*-carborane,

**5.** Emploi conforme à la revendication 4, dans lequel le carborane est un isomère 1,2-icosaédrique portant ou non un substituant.

**6.** Emploi conforme à la revendication 4, dans lequel le carborane est un isomère 1,7-icosaédrique portant ou non un substituant.

**7.** Emploi conforme à lune des revendications précédentes, dans lequel le composé est le chélate de manganèse et d'un ester tétrakis(carborane-carboxylate) de 2,4-bis(α,β-dihydroxy-éthyl)deutéro-porphyrine IX.

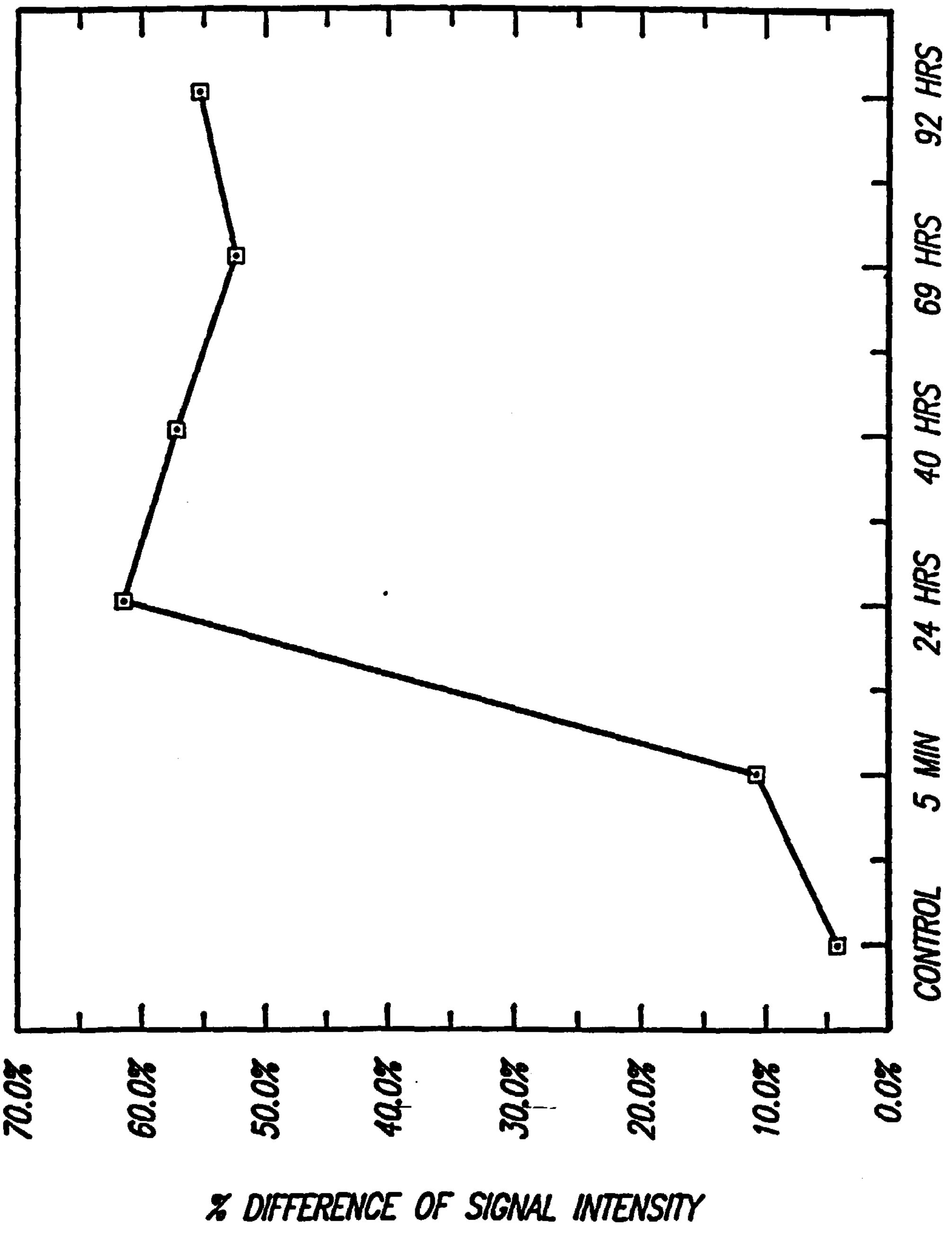
% DIFFERENCE OF SIGNAL INTENSITY
70.0%
60.0%
50.0%
40.0%
30.0%
20.0%
10.0%
0.0%
CONTROL
5 MIN
24 HRS
40 HRS
69 HRS
92 HRS

FIG. 1